# FASCICULE DE BREVET EUROPEEN

(11) **EP 1 938 793 B1**
(45) Date de publication et mention de la délivrance du brevet: **02.10.2013**
(21) Numéro de dépôt: 07122141.0
(22) Date de dépôt: 03.12.2007
(51) Int. Cl.: A61K 8/49, A61Q 19/02

(54) **Utilisation en cosmétique de la L-2-thiohistidine ou d'un de ses dérivés comme agent dépigmentant**
Einsatz von L-2-Thiohistidin oder eines seiner Derivate als Depigmentierungsmittel in der Kosmetik
Use in cosmetics of L-2-thiohistidine or one of its derivatives as a depigmentation agent

(30) Priorité: 29.12.2006 FR 0656063
(43) Date de publication de la demande: 02.07.2008
(73) Titulaire: LVMH RECHERCHE, 45800 St. Jean de Braye (FR)
(72) Inventeur: Andre, Patrice, 45170 Neuville aux Bois (FR); Marteau, Clarisse, 45370 Dry (FR); Renimel, Isabelle, 45470 Trainou (FR); Moreau, Marielle, 78770 Marcq (FR)
(74) Mandataire: Portal, Gérard

(56) Documents cités:
- EP-A- 0 483 426
- WO-A-95/34280
- WO-A-2006/124992
- US-A- 6 056 965
- DATABASE WPI Thomson Scientific, London, GB; AN 1981-95872D [52] XP002013753 & JP 56 147704 A (LOIRE KESHOHIN KK) 16 novembre 1981 (1981-11-16)
- TRADUCTION DE JP56147704

## Description

La présente invention concerne essentiellement l'utilisation, dans une composition cosmétique, de la L-2-thiohistidine ou d'un de ses dérivés comme agent dépigmentant ainsi qu'une méthode de soin cosmétique destinée à atténuer ou supprimer les taches pigmentaires de la peau et/ou à éclaircir le teint.

### ETAT DE LA TECHNIQUE

De nombreux agents dépigmentants sont connus dans l'état de la technique antérieure.

On connaît notamment par les documents JP 1973-044442et WO 2006/124992, une composition cosmétique à effet dépigmentant contenant de l'ergothionéine.

Les agents dépigmentants de l'art antérieur, tout en étant actifs, présentent divers inconvénients. Ils sont en effet souvent d'une formulation complexe et/ou d'une certaine toxicité, et/ou peuvent aussi présenter des effets secondaires indésirables, en particulier s'ils sont irritants ou allergisants.

La L-2-thiohistidine, aussi dénommée L-2-mercaptohistidine, correspond chimiquement à l'acide alpha amino-2,3 dihydro-2-thioxo-1H-imidazole-4 propanoïque et porte le numéro de registre CAS 2002-22-4.

Ce produit a déjà été utilisé dans le domaine de la cosmétique pour son effet déodorant.

Il est connu par le document japonais JP 56-147 704 l'emploi de composés à base de tropolone, sous forme de sels avec des aminoacides basiques parmi lesquels la thiohistidine. L'activité dépigmentante est attribuée aux composés à base de tropolone, la forme sel permettant de mieux solubiliser les composés de tropolone dans l'eau et d'accroître l'effet dépigmentant.

Dans ce document, il doit encore être observé qu'aucun des exemples ne vise la thiohistidine.

Dans ces conditions, il est clair que la thiohistidine n'a pas été testée et qu'il était donc non évident pour l'homme de l'art de découvrir son activité dépigmentante.

Les composés à base de tropolone sous forme de sels avec la thiohistidine sont exclus de la présente invention.

### BUTS DE L'INVENTION

La présente invention a pour but principal de fournir un nouvel agent dépigmentant ayant une très bonne efficacité dépigmentante et cela sans effets secondaires sensibles et sans toxicité sensible aux doses utilisées. La présente invention a pour but de fournir un tel nouvel agent dépigmentant d'une manière simple permettant une utilisation à l'échelle industrielle et cosmétique.

### RESUME DE L'INVENTION

La présente invention, selon un premier aspect, concerne l'utilisation dans une composition cosmétique, de la L-2-thiohistidine ou d'un sel ou d'un ester cosmétiquement acceptable de sa fonction acide, comme agent dépigmentant.

En particulier, une telle composition peut être utilisée pour dépigmenter la peau humaine.

Selon un deuxième aspect, la présente invention couvre aussi une méthode de soin cosmétique destinée à atténuer ou supprimer les taches pigmentaires de la peau et/ou à éclaircir le teint, caractérisée en ce qu'elle comprend l'application, sur au moins une zone concernée de la peau, d'une composition cosmétique contenant une quantité efficace de L-2-thiohistidine ou d'un sel ou d'un ester de sa fonction acide.

Selon un mode de réalisation particulier de l'invention, les sels de thiohistidine avec un composé à base de tropolone sont exclus en particulier les composés à base de tropolone tels que décrits dans le document japonais JP 56-147 104. Plus particulièrement, sont exclus les composés à base de tropolone cités tels que les sels de thiohistidine avec la tropolone, l'acide stipitatique, l'acide pubérulique, l'acide stipitique, l'acide pubérulonique, l'acide alpha-thuyaplicine, l'acide bêta -thuyaplicine, l'acide gamma-thuyaplicine, la nootkatine, la purpurogalline et la colchicéine.

Dans le cadre de chacun de ces aspects, on utilisera de préférence comme agent dépigmentant la L-2-thiohistidine.

Selon une variante, on pourra également utiliser un sel monovalent ou divalent de la fonction acide.

A titre de sels préférés de la fonction acide, on citera les sels de sodium, potassium, lithium, baryum, magnésium, calcium, ammonium, (HOCH₂CH₂)₃NH⁺, ou (C₂H₅)₃NH⁺.

A titre d'esters préférés de la fonction acide, on citera les esters d'alkyle ou d'hydroxyalkyle en C₁ à C₄, linéaire ou ramifié de la L-2-thiohistidine.

Dans le cadre de chacun de ces aspects, par quantité efficace, on entend la quantité minimum, qui est nécessaire pour obtenir une activité dépigmentante significative.

En général, la concentration préconisée en L-2-thiohistidine ou en son sel ou ester variera dans une large plage comprise entre 0,0001 et 1 % en poids de la composition finale.

Une plage préférée sera comprise entre 0,001 % et 0,1 % en poids.

D'autre part, on pourra utiliser la L-2-thiohistidine ou son sel ou ester en combinaison avec au moins un autre agent dépigmentant.

Dans le cadre de l'invention, on pourra aussi utiliser, dans la composition, au moins un autre agent cosmétiquement actif, tel qu'un agent anti-oxydant ou un agent apaisant.

On entend par agent anti-oxydant, une substance active capable de piéger les radicaux libres et/ou les substances réactives de l'oxygène.

Un tel agent anti-oxydant pourra notamment être choisi parmi les polyphénols, les dérivés séléniés comme l'ebselen, les dérivés de vitamine E ou encore l'idébénone.

On entend par agent apaisant, une substance active ayant un effet apaisant cutané permettant de prévenir et/ou de lutter contre les manifestations cutanées non-pathologiques d'origine inflammatoire, en particulier celles associées à la libération de cytokines, en particulier l'interleukine 8, et/ou de prostaglandines.

Un tel agent apaisant pourra notamment être choisi parmi les extraits de Scutellaria baicalensis, de Prunella vulgaris, de thé vert, ou encore être choisi parmi l'acide glycyrrhétinique et ses dérivés, en particulier le glycyrrhizinate d'ammonium ou de potasium et les stéaryl glycyrrhétinates.

D'autre part, comme cela est bien compréhensible également pour l'homme de l'art, on pourra ajouter tout excipient cosmétiquement acceptable pour fabriquer ladite composition cosmétique, en particulier naturellement des excipients ou agents cosmétiquement acceptables.

En outre, on pourra naturellement ajouter des exfoliants ainsi que divers agents conservateurs, parfums.

Par ailleurs, la composition cosmétique de l'invention peut être sous différentes formes, en particulier sous forme de lotions, de gels, d'émulsions, de crèmes, de poudres, de fonds de teint, ou de sticks.

L'homme du métier pourra aisément préparer la composition de l'invention par tout moyen classiquement utilisé pour préparer ce type de composition.

Par ailleurs, l'homme de l'art sait parfaitement sélectionner les zones de la peau ayant besoin d'être dépigmentées. Il s'agit généralement des zones de la peau du corps les plus exposées au rayonnement solaire ou les plus sensibles à l'environnement extérieur, tel que le visage, les membres supérieurs et inférieurs. Lorsqu'il s'agit des taches pigmentaires de vieillesse ou de taches associées à des hyperpigmentations localisées par hyper-activité mélanocytaires telles qu'un lentigo actinique, celles-ci peuvent naturellement se trouver dans n'importe quelle zone du corps.

D'autres buts, caractéristiques et avantages de l'invention apparaîtront clairement à la lumière de la description explicative, qui va suivre, faite en référence à plusieurs modes de réalisation actuellement préférés de l'invention donnés simplement à titre d'illustration et qui ne sauraient donc en aucune façon limiter la portée de l'invention.

L'exemple 1 ci-dessous est donné en référence aux figures 1 à 5 qui représentent respectivement, des clichés obtenus en microscopie optique d'épidermes traités par :
- figure 1 : la L-DOPA,
- figure 2 : l'acide kojique,
- figure 3 : l'ergothionéine,
- figure 4 : la L-2-thiohistidine à 50 µg/ml,
- figure 5 ; la L-2-thiohistidine à 150 µg/ml.

Dans les exemples, les pourcentages sont donnés en poids, la température est la température ambiante, la pression est la pression atmosphérique, sauf indication contraire.

Les exemples font partie intégrante de l'invention.

### EXEMPLE 1 SELON L'INVENTION

### Essais comparatifs démontrant l'activité dépigmentante de la L-2-thiohistidine

Dans cet exemple, on réalise un essai comparatif quantitatif, réalisé sur un épiderme séparé, destiné à montrer l'activité dépigmentante, de la L-2-thiohistidine en comparaison avec divers agents actifs dépigmentants antérieurement connus et notamment par rapport à l'acide kojique ou par rapport à l'ergothionéine, et encore comparativement à un échantillon témoin comprenant la DOPA oxydase.

La procédure de cet essai est similaire à celle décrite à l'exemple II du document LVMH RECHERCHE, FR-A-2,867,384, publié le 16 septembre 2005, auquel l'homme de l'art pourra utilement se reporter.

### Mode opératoire

Les épidermes provenant d'une plastie mammaire congelée d'une femme de 39 ans, référence P148AB39, sont séparés par incubation dans du NaBr 2N pendant 1 h 45 à 37°C. Ils ont été fixés dans un fixateur formolé, tamponné, rincé et mis en contact avec le mélange volume/volume de solution de L-DOPA/principe actif. Après incubation, ils ont été rincés et montés entre lames et lamelles avec du liquide de montage dénommé "mounting medium".

L'observation a été réalisée en microscopie optique avec un grossissement de 10.

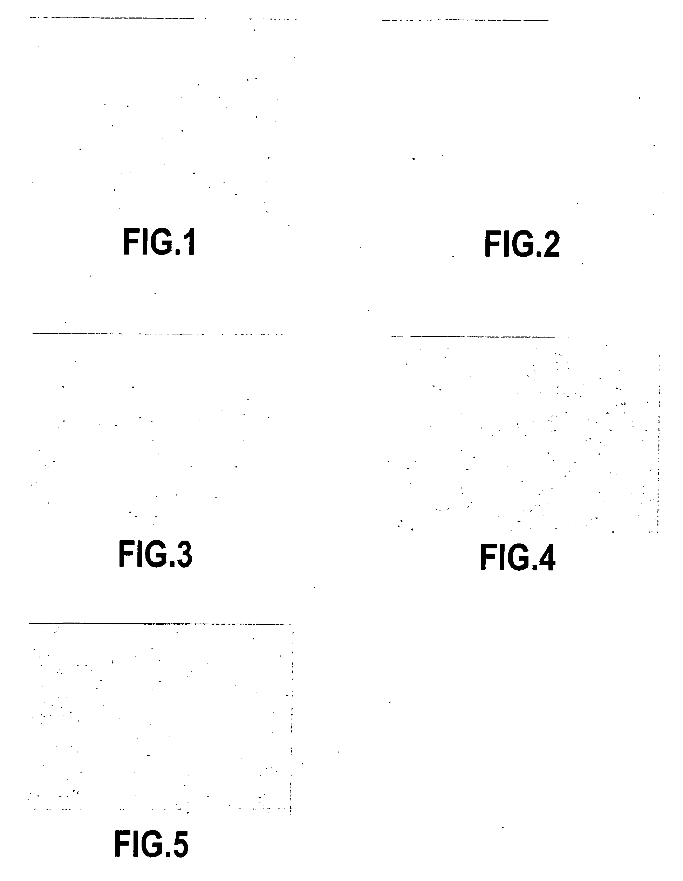

Les résultats observés sont les suivants :
a) avec le témoin DOPA, voir figure 1 annexée, les mélanocytes sont nettement visibles et bien pigmentés ;
b) avec l'acide kojique à la concentration de 150 µg/ml, comme agent dépigmentant comparatif, voir figure 2, on constate que les mélanocytes ne sont que légèrement visibles et qu'il y a donc une très bonne activité dépigmentante. L'activité dépigmentante est ici proche de 75 %;
c) avec l'ergothionéine à la concentration de 50 µg/ml, comme agent dépigmentant comparatif, figure 3, on observe que les mélanocytes sont assez bien visibles et toujours assez bien pigmentés. On observe une légère activité dépigmentante proche de 45 % ;
d) avec la L-2-thiohistidine, à la concentration de 50 µg/ml selon l'invention, figure 4, on constate que les mélanocytes sont moins visibles qu'avec l'ergothionéine à la même concentration, figure 3. L'activité dépigmentante, ici de 60 %, est donc bonne.
e) avec la L-2-thiohistidine utilisée comme agent dépigmentant à la concentration de 150 µg/ml, figure 5, on observe que les mélanocytes sont maintenant très peu visibles car très peu pigmentés. L'activité dépigmentante est ici de 75 % soit une très bonne activité dépigmentante.
L'activité dépigmentante visible sur les figures 1 à 5 a été quantifiée statistiquement par décompte des grains de mélanine sur une série de 3 essais, les valeurs sont reprises dans le tableau ci-après, dans lequel les valeurs obtenues pour le test de Student sont ici précisées pour l'ergothionéine versus la L-2-thiohistidine et l'acide kojique versus la L-2-thiohistidine à concentration égale. Le test de Student est dit significatif si la valeur obtenue est inférieure à 0,05.

### Conclusions

Dans les conditions expérimentales, on constate qu'à la concentration de 150 µg/ml, la L-2-thiohistidine a une très bonne activité dépigmentante, similaire à celle de l'acide kojique préalablement connu et souvent utilisé comme référence expérimentale en terme d'activité mais dont les effets secondaires sont par ailleurs bien connus.

On constate également que l'utilisation de la L-2-thiohistidine selon l'invention permet une meilleure activité dépigmentante que l'ergothionéine et une activité similaire à celle de l'acide kojique à concentration comparable, ce qui démontre l'intérêt industriel et cosmétique de l'utilisation dans une composition cosmétique de la L-2-thiohistidine comme agent dépigmentant.

On va maintenant décrire ci-après divers exemples de compositions cosmétiques utilisant la L-2-thiohistidine comme agent dépigmentant, dans lesquels toutes les valeurs sont données en pourcentages en poids par rapport au poids total de la composition, sauf indication contraire.

### EXEMPLE 2 SELON L'INVENTION

**Composition cosmétique selon l'invention sous forme d'un gel dépigmentant**

| 1°. Agent dépigmentant | |
|---|---|
| L-2-thiohistidine | 0.5 |

| 2°. Excipients | |
|---|---|
| Glycol | 3 |
| Polyacrylamide/C13-14 isoparaffine/laureth-7 (Sepigel 305) | 3 |
| Huile de ricin hydrogénée (Cremophor CO 60) | 2 |
| Polyéthylèneglycol (Pluriol E 1505) | 1.5 |
| Conservateur | 0.5 |
| Concentré de parfum | 0.3 |
| Eau | 89.2 |

La procédure de fabrication de la composition cosmétique ci-dessus est tout à fait classique. On mélange tout d'abord les différents composants des excipients en l'absence de l'agent gélifiant constitué ici par le Sepigel 305 et ensuite on ajoute l'agent dépigmentant et enfin, l'agent gélifiant.

La composition obtenue se présente sous la forme d'un gel à activité dépigmentante. On peut appliquer ce gel sur les zones de la peau à dépigmenter deux fois par jour pendant deux semaines, ce qui permet de réaliser une dépigmentation efficace des zones de la peau concernées.

### EXEMPLE 3 SELON L'INVENTION

**Composition cosmétique selon l'invention sous forme d'une émulsion dépigmentante**

| 1°. Agent dépigmentant | |
|---|---|
| L-2-thiohistidine | 0.01 |

| 2°. Excipients | |
|---|---|
| Stéareth-21 (Brij 721) | 2.5 |
| Glycéryl stéarate (Tegin 90) | 1.1 |
| Alcool stéarylique | 5 |
| Tricaprat / caprilate glycérol | 20 |
| Butylène glycol | 3 |
| Glycérol | 2 |
| Conservateur | 0.5 |
| Concentré de parfum | 0.5 |
| Eau | qsp 100 |

La procédure de fabrication de cette composition cosmétique est similaire à celle de l'exemple 2, si ce n'est qu'ici, on mélange tout d'abord à chaud tous les constituants, à l'exception de l'eau et de la L-2-thiohistidine qui sont ajoutés à froid par la suite.

La L-2-thiohistidine est de préférence ajoutée dans la phase aqueuse. On mélange vigoureusement sous agitation pour former une émulsion stable. Ensuite, on conditionne en obtenant ainsi la composition cosmétique sous forme d'une émulsion dépigmentante.

Cette émulsion peut être appliquée de manière similaire au gel dépigmentant de l'exemple 2 pour obtenir l'effet dépigmentant recherché.

### EXEMPLE 4 SELON L'INVENTION

**Composition cosmétique selon l'invention sous forme de lotion dépigmentante**

| 1°. Agent dépigmentant | |
|---|---|
| L-2-thiohistidine | 0.001 |

| 2°. Excipients | |
|---|---|
| Butylène glycol | 3 |
| EDTA | 0.1 |
| Solubilisant | 1 |
| Concentré de parfum | 0.3 |
| Alcool | 5 |
| Eau | qsp |

La procédure de fabrication de cette composition sous forme de lotion dépigmentante est particulièrement simple. On mélange tout d'abord l'ensemble des excipients auxquels on ajoute ensuite l'agent dépigmentant constitué par la L-2-thiohistidine sous agitation, pour obtenir une solution stable sous forme de lotion dépigmentante.

### EXEMPLE 5 SELON L'INVENTION

**Composition cosmétique selon l'invention sous forme de poudre pour éclaircir le teint**

| 1°. Agent dépigmentant | |
|---|---|
| L-2-thiohistidine | 0.005 |

| 2°. Excipients | |
|---|---|
| Talc | 20 |
| Mica | 20 |
| Séricite | 20 |
| Pigments | 8 |
| Poudre organique (Nylon) | 20 |
| silice | 91.005 |
| Huile minérale ou silicone | 3 |

La procédure de fabrication de cette composition cosmétique sous forme de poudre pour éclaircir le teint est particulièrement simple. On mélange sous agitation l'ensemble des excipients et de l'agent dépigmentant jusqu'à obtenir une poudre uniforme constituant la composition cosmétique recherchée.

On peut appliquer cette composition cosmétique sous forme de poudre pour éclaircir le teint chaque matin, ou le soir, selon la préférence de la personne.

### EXEMPLE 6 SELON L'INVENTION

**Composition cosmétique selon l'invention sous forme de fond de teint éclaircissant**

| 1°. Agent dépigmentant | |
|---|---|
| L-2-thiohistidine | 0.0001 |

| 2°. Excipients | |
|---|---|
| Polyglycéryl-4 isostéarate/cetyldiméthicone/hexyllaurate | 5.1 |
| Cyclopentasiloxane/Cyclohexasiloxane | 5.0 |
| Cétyl diméthicone | 1.0 |
| Caprylic/capric triglycérides | 2.2 |
| Octyl Stéarate | 1.4 |
| Huile minérale | 6.5 |
| Huile de ricin Hydrogénée | 1.2 |
| Cire d'abeilles | 0.8 |
| Polyméthacrylate de méthyle | 1.1 |
| Fe₂O₃ | 0.45 |
| TiO₂ | 5.2 |
| NaCl | 0.6 |
| Concentré de parfum | 0.1 |
| Eau | qsp |

On procède ici comme dans l'exemple 3 et on mélange l'ensemble des composants en plusieurs étapes, comme cela est bien compréhensible à l'homme de l'art et on ajoute en phase finale la L-2-thiohistine à froid. On réalise une vigoureuse agitation jusqu'à obtenir un mélange uniforme formant la composition cosmétique sous forme de fond de teint éclaircissant.

Ce fond de teint éclaircissant peut être appliqué sur les zones de la peau à dépigmenter quotidiennement, au moins une fois par jour, jusqu'à obtention de l'éclaircissement des zones pigmentées.

Ainsi, on constate que la présente invention permet d'aboutir très efficacement, et d'une manière simple, à un effet dépigmentant ou éclaircissant des zones de la peau pigmentées. L'invention permet en outre de réaliser toutes les formes de compositions cosmétiques possibles.

**Tableau**

| | Essai 1 | Essai 2 | Essai 3 | moyenne | écart type | student/témoin | student/ergothionéine | student/acide kojique |
|---|---|---|---|---|---|---|---|---|
| Témoin | 409 | 430 | 433 | 424 | 13,08 | | | |
| Kojic 150 µg | 124 | 137 | 138 | 133 | 7,81 | 4,97363E-06 | | |
| Ergothioneine 50 µg | 194 | 210 | 190 | 198 | 10,58 | 2,02171E-05 | | |
| Thiohistidine 50µg | 162 | 178 | 176 | 172 | 8,72 | 9,99899E-06 | 0,000331269 | |
| Thiohistidine 150µg | 102 | 115 | 116 | 111 | 7,81 | 3,71899E-06 | | 0,026057433 |

## Revendications

1. Utilisation non-thérapeutique de la L-2-thiohistidine ou d'un sel ou d'un ester cosmétiquement acceptable de sa fonction acide, comme agent dépigmentant dans une composition cosmétique.

2. Utilisation selon la revendication 1, de la L-2-thiohistidine ou d'un sel ou d'un ester cosmétiquement acceptable, comme agent dépigmentant de la peau humaine.

3. Utilisation selon la revendication 1 ou 2, **caractérisée en ce que** ledit agent dépigmentant est la L-2-thiohistidine.

4. Utilisation selon la revendication 1 ou 2, **caractérisée en ce que** ledit agent dépigmentant est un sel de la fonction acide de la L-2-thiohistidine, en particulier un sel de sodium, potassium, lithium, baryum, magnésium, calcium, ammonium, (HOCH₂CH₂)₃NH⁺, ou (C₂H₅)₃NH⁺.

5. Utilisation selon la revendication 1 ou 2, **caractérisée en ce que** ledit agent dépigmentant est un ester d'alkyle ou d'hydroxyalkyle en C₁ à C₄, linéaire ou ramifié de la L-2-thiohistidine.

6. Utilisation selon l'une quelconque des revendications 1 à 5, **caractérisée en ce que** ladite composition cosmétique contient entre 0,0001 % et 1 % en poids de L-2-thiohistidine ou de son sel ou ester cosmétiquement acceptable.

7. Utilisation selon la revendication 6, **caractérisée en ce que** ladite composition cosmétique contient entre 0,001 % et 0,1 % en poids de L-2-thiohistidine ou de son sel ou ester cosmétiquement acceptable.

8. Utilisation selon l'une quelconque des revendications 1 à 7, **caractérisée en ce que** ladite composition cosmétique contient en outre au moins un autre agent dépigmentant.

9. Utilisation selon l'une quelconque des revendications 1 à 8, **caractérisée en ce que** ladite composition cosmétique contient en outre au moins un autre agent cosmétiquement actif, tel qu'un agent anti-oxydant ou un agent apaisant.

10. Méthode de soin cosmétique, non-thérapeutique destinée à atténuer ou supprimer les taches pigmentaires de la peau et/ou à éclaircir le teint, **caractérisée en ce qu'**elle comprend l'application, sur au moins une zone concernée de la peau, d'une composition cosmétique telle que définie dans l'une des revendications 1 à 9, contenant une quantité efficace de L-2-thiohistidine ou d'un de ses sels ou esters cosmétiquement acceptables de sa fonction acide.

## Patentansprüche

1. Nicht-therapeutische Verwendung von L-2-Thiohistidin oder von einem kosmetisch akzeptablen Salz oder Ester seiner Säurefunktion, als Degmentierungsmittel in einer kosmetischen Zusammensetzung.

2. Verwendung nach Anspruch 1, von L-2-Thiohistidin oder von einem kosmetisch akzeptablen Salz oder Ester als Depigmentierungsmittel der menschlichen Haut.

3. Verwendung nach Anspruch 1 oder 2, **dadurch gekennzeichnet, daß** das Depigmentierungsmittel L-2-Thiohistidin ist.

4. Verwendung nach Anspruch 1 oder 2, **dadurch gekennzeichnet, daß** das Depigmentierungsmittel ein Salz der Säurefunktion von L-2-Thiohistidin, insbesondere ein Natrium-, Kalium-, Lithium-, Barium-, Magnesium-, Kalzium-, Ammoniumsalz, (HOCH₂CH₂)₃NH⁺ oder (C₂H₅)₃NH⁺ ist.

5. Verwendung nach Anspruch 1 oder 2, **dadurch gekennzeichnet, daß** das Depigmentierungsmittel ein linearer oder verzweigter C₁- bis C₄-Alkyl- oder Hydroxyalkylester von L-2-Thiohistidin ist.

6. Verwendung nach einem der Ansprüche 1 bis 5, **dadurch gekennzeichnet, daß** die kosmetische Zusammensetzung zwischen 0,0001 Gew.-% und 1 Gew.-% L-2-Thiohistidin oder seines kosmetisch akzeptablen Salzes oder Esters enthält.

7. Verwendung nach Anspruch 6, **dadurch gekennzeichnet, daß** die kosmetische Zusammensetzung zwischen 0,001 Gew.-% und 0,1 Gew.-% L-2-Thiohistidin oder seines kosmetisch akzeptablen Salzes oder Esters enthält.

8. Verwendung nach einem der Ansprüche 1 bis 7, **dadurch gekennzeichnet, daß** die kosmetische Zusammensetzung ferner wenigstens ein weiteres Depigmentierungsmittel enthält.

9. Verwendung nach einem der Ansprüche 1 bis 8, **dadurch gekennzeichnet, daß** die kosmetische Zusammensetzung ferner wenigstens eine weitere kosmetisch aktive Substanz, wie ein Antioxidans oder ein linderndes Mittel enthält.

10. Nicht-therapeutisches kosmetisches Pflegeverfahren, das dazu bestimmt ist, Pigmentflecken der Haut zu mildern oder zu beseitigen und/oder den Teint aufzufrischen, **dadurch gekennzeichnet, daß** es das Auftragen einer kosmetischen Zusammensetzung, wie sie in einem der Ansprüche 1 bis 9 definiert ist, die eine wirkungsvolle Menge von L-2-Thiohistidin oder von einem seiner kosmetisch akzeptablen Salze oder Ester seiner Säurefunktion enthält, auf wenigstens einen betroffenen Bereich der Haut umfaßt.

## Claims

1. Non-therapeutic use of L-2-thiohistidine, or a cosmetically acceptable salt or ester of its acid group, as a depigmenting agent in a cosmetic composition.

2. Use according to claim 1 of L-2-thiohistidine or a cosmetically acceptable salt or ester as a depigmenting agent for human skin.

3. Use according to claim 1 or 2 wherein said depigmenting agent is L-2-thiohistidine.

4. Use according to claim 1 or 2 wherein said depigmenting agent is a salt of the acid group of L-2-thiohistidine, particularly a sodium, potassium, lithium, barium, magnesium, calcium, ammonium, (HOCH₂CH₂)₃NH⁺ or (C₂H₅)₃NH⁺ salt.

5. Use according to claim 1 or 2 wherein said depigmenting agent is a linear or branched C₁ to C₄ alkyl or hydroxyalkyl ester of L-2-thiohistidine.

6. Use according to any one of claims 1 to 5 wherein said cosmetic composition contains between 0.0001 % and 1% by weight of L-2-thiohistidine or its cosmetically acceptable salt or ester.

7. Use according to claim 6 wherein said cosmetic composition contains between 0.001% and 0.1% by weight of L-2-thiohistidine or its cosmetically acceptable salt or ester.

8. Use according to any one of claims 1 to 7 wherein said cosmetic composition also contains at least one other depigmenting agent.

9. Use according to any one of claims 1 to 8 wherein said cosmetic composition also contains at least one other cosmetically active agent, such as an antioxidant or a soothing agent.

10. A method of non-therapeutic cosmetic care for toning down or eliminating pigment spots on the skin and/or lightening the complexion, which comprises the application, to at least one appropriate area of skin, of a cosmetic composition as defined in one of claims 1 to 9, containing an effective amount of L-2-thiohistidine or one of its cosmetically acceptable salts or esters of its acid group.
